# EUROPEAN PATENT SPECIFICATION

(11) **EP 4 083 207 B1**
(45) Date of publication and mention of the grant of the patent: **25.03.2026**
(21) Application number: 20904326.4
(22) Date of filing: 25.12.2020
(51) Int. Cl.: C12N 15/113, A01H 1/00

(54) **METHOD FOR SUPPRESSING METHYLATION OF TARGET DNA IN PLANT**
VERFAHREN ZUR UNTERDRÜCKUNG DER METHYLIERUNG VON ZIEL-DNA IN PFLANZEN
PROCÉDÉ DE SUPPRESSION DE LA MÉTHYLATION D'UN ADN CIBLE DANS UNE PLANTE

(30) Priority: 26.12.2019 JP 2019237374
(43) Date of publication of application: 02.11.2022
(73) Proprietor: National Institute of Advanced Industrial Science and Technology, Chiyoda-ku Tokyo 100-8921 (JP)
(72) Inventor: ATSUMI, Go, Sapporo-shi, Hokkaido 062-8517 (JP); MATSUMURA, Takeshi, Sapporo-shi, Hokkaido 062-8517 (JP); MASUTA, Chikara, Sapporo-shi, Hokkaido 060-0808 (JP); INUKAI, Tsuyoshi, Sapporo-shi, Hokkaido 060-0808 (JP); MATSUNAGA, Wataru, Sapporo-shi, Hokkaido 060-0808 (JP); ISODA, Reika, Sapporo-shi, Hokkaido 060-0808 (JP)
(74) Representative: Henderson, Helen Lee
(86) International application number: PCT/JP2020/048997
(87) International publication number: WO 2021/132669

(56) References cited:
- WO-A1-2004/078971
- WO-A1-2020/022461
- RUIQIANG YE ET AL: "Cytoplasmic Assembly and Selective Nuclear Import ofARGONAUTE4/siRNA Complexes", MOLECULAR CELL, ELSEVIER, AMSTERDAM, NL, vol. 46, no. 6, 12 April 2012 (2012-04-12), pages 859 - 870, XP028502148, ISSN: 1097-2765, [retrieved on 20120417], DOI: 10.1016/J.MOLCEL.2012.04.013
- ANONYMOUS: "2.1.3-2. Development of expression control technology by specific demethylation of target gene, document 7-1 Development of Production Techniques for Highly Functional Biomaterials Using Plant and Other Organism Smart Cells", MATERIALS TECHNOLOGY AND NANOTECHNOLOGY DEPARTMENT, 1 July 2018 (2018-07-01), XP055834768, Retrieved from the Internet <URL:https://www.nedo.go.jp/content/100883897.pdf>
- MATSUMURA KEN: "Deployment to high productivity of compound by new and old plant biotechnology", DEVELOPMENT OF PRODUCTION TECHNIQUES FOR HIGHLY FUNCTIONAL BIOMATERIALS USING PLANT AND OTHER ORGANISM SMART CELLS, 14 November 2016 (2016-11-14), XP055834761, Retrieved from the Internet <URL:https://www.nedo.go.jp/content/100805433.pdf>
- W MATSUNANGA , R ISODA , T INUKAI , T MATSUMURA , C MASUTA: "A technique to reduce dna methy lat ion in a sequence-specific manner by using a ribozyme-expressing cucumber mosaic virus vector", PHYTOPATHOLOGY, AMERICAN PHYTOPATHOLOGICAL SOCIETY, US, vol. 108, no. 10S, 15 October 2018 (2018-10-15), US, pages S1.15 - S1.16, XP009525903, ISSN: 0031-949X

## Description

### FIELD

The present invention relates to a method for inhibiting methylation of target DNA in plants, and an expression system for accumulation of a plant-derived functional component.

### BACKGROUND

Gene expression in plants is regulated by DNA methylation and chemical modification of histones, which are collectively known as epigenetic control. It occurs by the mechanism of RNA silencing, with RNA silencing being largely divided into two types: post-transcriptional gene silencing (PTGS) and transcriptional gene silencing (TGS). Plants depend on epigenetic control for regulation of levels of accumulation of functional components, and techniques for freely manipulating epigenetics are necessary to cause accumulation of useful components at high levels in plants. However, epigenetic control has been considered too complex for specifically removing methylation from target DNA.

The technology for directing demethylation to specific DNA sequences is virtually non-existent, with only one technique for directing demethylation using dCAS-fused TET1 finally being reported in recent years (NPL 1). However, using dCAS-fused TET1 to direct demethylation requires recombinant technology, which means that demethylation cannot be directed in a convenient and rapid manner.

### [CITATION LIST]

### [PATENT LITERATURE]

[PTL 1] International Patent Publication No. WO2014/129560

### [NON PATENT LITERATURE]

[NPL 1] Gallego-Bartolome J et al., Proceedings of the National Academy of Sciences of the United States of America 55, E2125-E2134 (2018)
[NPL 2] Gallusci P et al., Trends in Plant Science 22,610-623 (2017)
[NPL 3] Matzke M. A and Mosher R. A, Nature Reviews Genetics 15,394-408 (2014)
[NPL 4] Philips J. G et al., PloS one, 12(2), e0171311 (2017)
[NPL 5] Otagaki S et al., Plant Biotechnology 23,259-265 (2006)
[NPL 6] Ye R et al., Molecular Cell 46,859-870 (2012)
[NPL 7] Anonymous, "2.1.3.2. Development of expression control technology by specific demethylation of target gene, document 7-1 Development of Production Techniques for Highly Functional Biomaterials Using Plant and Other Organism Smart Cells", Materials Technology and Nanotechnology Department (1 July 2018),124-126

### SUMMARY

### [TECHNICAL PROBLEM]

It is an object of the present invention to create plants with desired traits by conveniently and rapidly inhibiting methylation of target DNA in plants, either using recombinant technology or without using recombinant technology.

### [SOLUTION TO PROBLEM]

As a result of avid research on the object stated above, the present inventors have found, surprisingly, that in an RNA-directed DNA methylation mechanism, inhibiting binding of scaffold RNA produced by transcription of target DNA with siRNA-AGO4 complex allows methylation of the target DNA in the plants to be specifically inhibited.

Specifically, the present invention is as follows.
[1] A method for inhibiting methylation of a target DNA in a plant cell, comprising a step of inhibiting binding of a scaffold RNA produced by transcription of the target DNA with an siRNA-AGO4 complex, in an RNA-directed DNA methylation mechanism, wherein the step of inhibiting binding of the scaffold RNA with the siRNA-AGO4 complex is accomplished by introducing into the plant cell a short dummy RNA, wherein the short dummy RNA comprises i) a sequence complementary to the siRNA incorporated in the siRNA-AGO4 complex, or ii) a sequence complementary to the scaffold RNA, and wherein the length of the scaffold RNA is 20 bp to 50 bp.
[2] The method according to [1] above, wherein the short dummy RNA comprises a nucleotide sequence having at least 80% or greater, 85% or greater, 90% or greater, 92% or greater, 94% or greater, 95% or greater, 96% or greater, 97% or greater, 98% or greater or 99% or greater identity with the target DNA.
[3] The method according to any one of [1] or [2] above, wherein the target DNA is a promoter that controls a gene expressing a desired trait for plant cell. [4] The method according to [3] above, wherein the gene that expresses the desired trait is a gene coding for the amino acid sequence of an enzyme that controls synthesis or accumulation of a plant-derived functional component.
[5] The method according to any one of [1] to [4] above, wherein the short dummy RNA is introduced into the plant cell by a plant virus vector method, an agroinfiltration method, a magnICON^{®} system or a particle gun method.
[6] The method according to any one of [1] to [5] above, wherein the plant cell is a non-isolated cell of a plant body.
[7] The method according to any one of [1] to [5] above, wherein the plant cell is a cultured cell.
[8] A method for creating a plant with a desired trait, comprising a step of inhibiting methylation of a DNA involved in expression of the desired trait for the plant by using the method according to any one of [1] to [7].
[9] A method for producing a plant-derived functional component, comprising steps of inhibiting methylation of DNA involved in synthesis or accumulation of a plant-derived functional component by using the method according to any one of [1] to [7] above, thereby causing accumulation of the functional component in the plant cells, and recovering the functional component from the plant cell.
[10] An expression system for accumulation of a plant-derived functional component, comprising: (A) a plant body or a plant cell that produces a scaffold RNA produced by transcription of a DNA involved in synthesis or accumulation of the plant-derived functional component, and siRNA-AGO4 complex, in an RNA-directed DNA methylation mechanism, and
   (B) a short dummy RNA comprising a sequence complementary to the siRNA incorporated in the siRNA-AGO4 complex, or a sequence complementary to the scaffold RNA, and wherein the length of the scaffold RNA is 20 bp to 50 bp.
[11] The expression system according to [10] above wherein the short dummy RNA comprises a nucleotide sequence having at least 80% or greater, 85% or greater, 90% or greater, 92% or greater, 94% or greater, 95% or greater, 96% or greater, 97% or greater, 98% or greater or 99% or greater identity with the DNA involved in synthesis or accumulation of the plant-derived functional component.

### [ADVANTAGEOUS EFFECTS OF INVENTION]

According to the present invention it is possible to inhibit methylation of target DNA in plants, thereby allowing convenient and rapid specific control of TGS of the target DNA. It is thereby possible to obtain plants with desired traits, thus greatly contributing to production of useful proteins or increased accumulation levels of functional components in plants.

### BRIEF DESCRIPTION OF DRAWINGS

Fig. 1 shows a DNA methylation model in an RdDM pathway.
Fig. 2 shows a DNA demethylation model based on capture of siRNA-AGO4 complex by short dummy RNA.
Fig. 3 shows a DNA demethylation model based on blocking binding between scaffold RNA and siRNA-AGO4 by short dummy RNA.
Fig. 4 shows the sequence of the 35S promoter as the target of short dummy RNA, and the sequence of a short dummy RNA construct (SD-51) created to target the portion where scaffold RNA is abundantly detected (-51 to -76).
Fig. 5 shows a fluorescent image of a GFP expression-restored individual in first generation N. benthamiana expressing SD-51 by CMV-A1 vector (indicated as SD-51 in the drawing), compared to the control (16c and 208RED).
Fig. 6 shows a fluorescent image of a GFP expression-restored individual (35 days after seeding) in second generation N. benthamiana, expressing SD-51 by CMV-A1 vector.
Fig. 7 shows a schematic view for categorization of phenotypes of GFP expression in N. benthamiana expressing SD-51 by CMV-A1 vector.

### DESCRIPTION

### [1. Background]

Gene expression in plants is regulated by epigenetic control. The term "epigenetics" refers to changes in gene function and structure without alteration in the DNA sequence, which are still transmitted through cell division. DNA methylation is known as one type of mechanism of epigenetics (NPL 2). The reaction includes either methyl group-addition reaction at the 5-position carbon atom of the pyrimidine ring of cytosine or the 6-position nitrogen atom of the purine ring of adenine, but it is believed that gene expression in plants is mainly controlled by methylation of cytosine. Specifically, methylation and demethylation of cytosine in DNA switches gene expression on and off without changing the nucleotide sequence information itself. Suppression of gene expression by DNA methylation is known as transcriptional gene silencing (TGS).

The present invention is based on the foundational concept that artificially controlling epigenetic regulation in plants allows alteration to desired plant phenotypes without gene recombination.

Great advances have been made in understanding DNA methylation in *Arabidopsis thaliana* as a model plant. In plants, methylation takes place at the CpG, CpHpG and CpHpH sites (H representing a nucleotide other than guanine), and DRM2, MET1, CMT3 and CMT2 are known as the main DNA methyltransferase enzymes that transfer and covalently bond methyl groups to DNA. DNA methyltransferases are currently classified into the two types of: *de novo* enzymes that create new methyl labeling on DNA, and: maintenance enzymes that recognize methylation locations on the DNA parent strand and transmit the new methylation to the daughter chain after DNA replication, and only DRM2 is known to be a *de novo* DNA methyltransferase. While it is not clearly understood how the locations for *de novo* DNA methylation are determined by cells, previous evidence suggests that the mechanism of RNA-directed DNA methylation (RdDM) is involved for most locations.

As mentioned above, the present invention is based on the finding that in an RNA-directed DNA methylation mechanism, inhibiting binding of scaffold RNA produced by transcription of target DNA with siRNA-AGO4 complex allows methylation of the target DNA in the plants to be specifically inhibited. The RNA-directed DNA methylation mechanism used for the present invention will therefore be explained first.

### [RdDM mechanism]

The RdDM mechanism is a mechanism by which methylation of target DNA is induced via the DNA region that is the target of methylation (target DNA) and low molecular double stranded RNA (siRNA) at least partially having the same nucleotide sequence. DNA methylation can be mediated by siRNA binding to Argonaut 4 (AGO4; NPL 6).

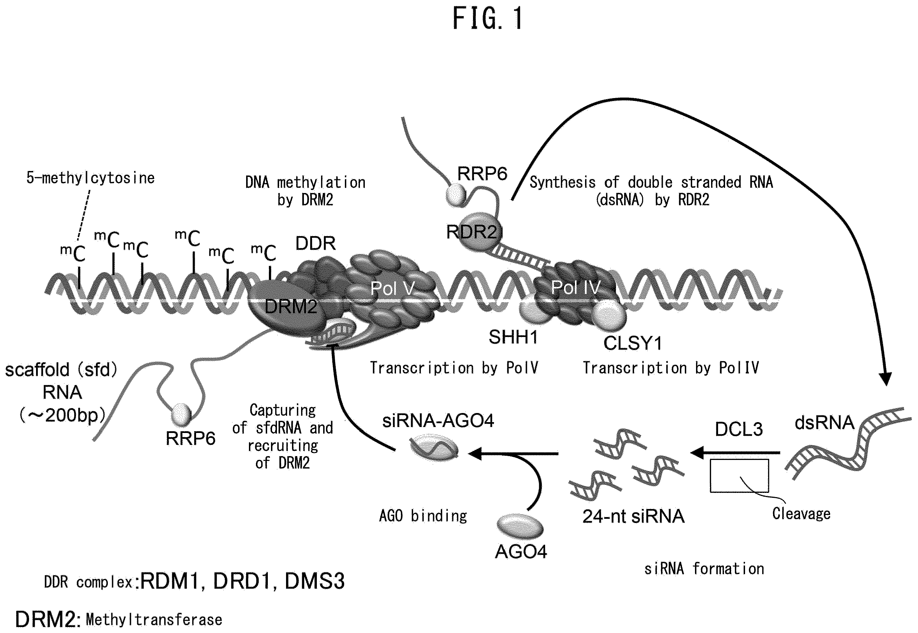

In the RdDM mechanism, as shown in Fig. 1, first PolIV is recruited to the methylation target genome region and the RNA of the target region is transcribed. The PolIV transcription product is converted to double-stranded RNA by RNA-Dependent RNA Polymerase 2 (RDR2), and cleaved into 24-nucleotide siRNA by the RNaseIII-like enzyme Dicer-like 3 (DCL3) (NPL 3). The siRNA is modified by methylation at the 3'-end by HUA Enhancer 1 (HEN1) and then taken up into AGO4 to form a silencing effector complex (hereunder referred to as "siRNA-AGO4 complex"). Complementary siRNA taken up into the siRNA-AGO4 complex forms base pairs with RNA transcribed by DNA-dependent RNA polymerase V (PolV) (hereunder referred to as "scaffold RNA"), thereby recruiting the complex. Domains Rearranged Methyltransferase (DRM2), a de novo methyltransferase in the DNA region corresponding to the siRNA is then recruited via siRNA-AGO4 complex, and the DNA is methylated. RNA-Directed DNA Methylation 1 (RDM1), Defective In RNA-Directed DNA Methylation 1 (DRD1) and Defective In Meristem Silencing 3 (DMS3), which are able to bind AGO4, DRM2 and methylated DNA, are thought to play an important role in recruiting.

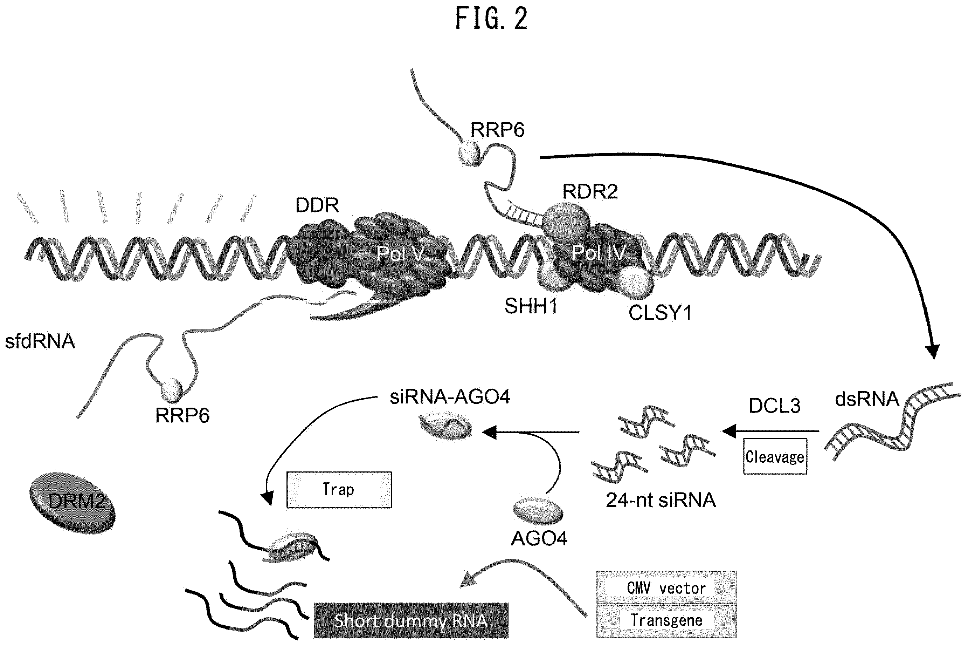

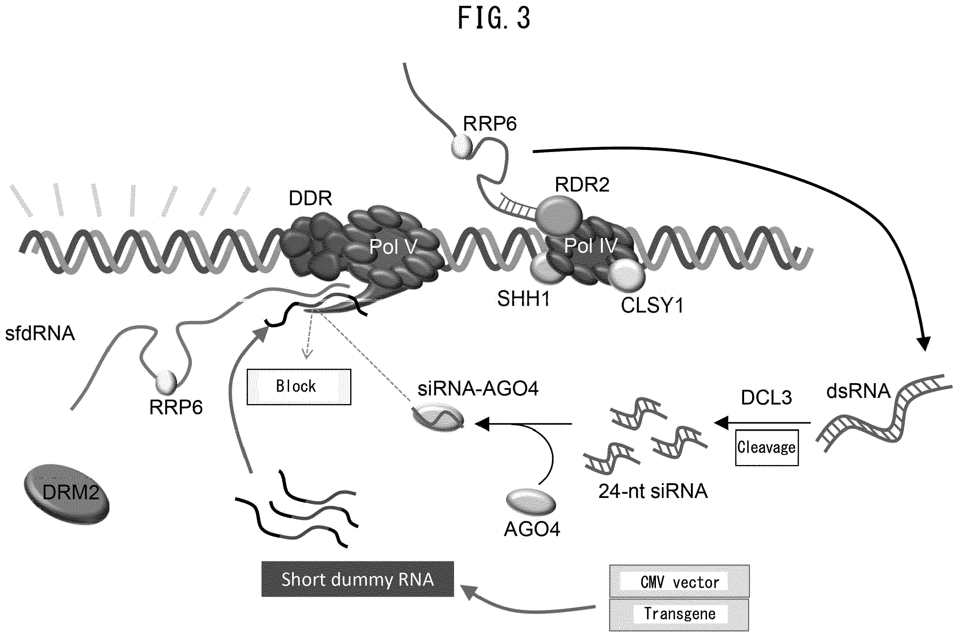

The RNA transcribed by PolIV and PolV from the target DNA (which have essentially the same sequences since they are transcribed from the same region, although the RNA transcribed by PolV is referred to specifically as "scaffold RNA"), have the function of bringing methyl group transferase DRM2 to the target DNA via binding with siRNA-AGO4 complex. Therefore, the siRNA-AGO4 complex is either captured by short RNA comprising a sequence complementary to the siRNA incorporated in the siRNA-AGO4 complex which has the role of recruiting DRM2 to the target sequence (hereunder referred to as "short dummy RNA") (Fig. 2), or the short dummy RNA is bound to the scaffold RNA beforehand to block binding with siRNA-AGO4, (Fig. 3), thereby preventing recruit of DRM2 to the target sequence, and thus allowing DNA methylation to be inhibited. In contrast, other expression control systems have required scaffold RNA to be removed (NPL 7).

### [2. Method for inhibiting methylation of target DNA in plant cells]

Described herein is a method for inhibiting methylation of target DNA in plant cells, wherein the method comprises inhibiting binding of scaffold RNA produced by transcription of the target DNA with siRNA-AGO4 complex, in an RNA-directed DNA methylation mechanism.

By inhibiting binding between siRNA-AGO4 complex and scaffold RNA that is the factor in DNA methylation that suppresses expression of a desired trait in a plant, recruiting of DRM2 to the region of the DNA corresponding to the siRNA is inhibited, thereby specifically inhibiting methylation of the target DNA in the plant. Inhibition of binding of scaffold RNA with siRNA-AGO4 complex can be accomplished, for example, by introducing into the plant cells short dummy RNA comprising a sequence complementary to the siRNA incorporated in the siRNA-AGO4 complex, or by introducing into the plant cells short dummy RNA comprising a sequence complementary to at least a portion of the scaffold RNA.

The term "short dummy RNA" used herein refers to RNA that is short enough to avoid eliciting RdDM, and that can bind to at least a portion of the siRNA incorporated into siRNA-AGO4 complex and/or scaffold RNA. The short dummy RNA preferably comprises a nucleotide sequence having at least 80% or greater, 85% or greater, 90% or greater, 92% or greater, 94% or greater, 95% or greater, 96% or greater, 97% or greater, 98% or greater or 99% or greater identity with the target DNA. Since such a sequence is complementary to at least a portion of the siRNA incorporated in the siRNA-AGO4 complex and/or the scaffold RNA, the short dummy RNA can prevent recruiting of DRM2 to the target sequence in a DNA demethylation model based on capturing the siRNA-AGO4 complex (Fig. 2), and/or in a DNA demethylation model based on blocking binding between the scaffold RNA and siRNA-AGO4 by short dummy RNA (Fig. 3), thereby inhibiting methylation of the target DNA. As explained above, RNA transcribed by PolIV and RNA transcribed by PolV from target DNA have essentially the same sequence since they are transcribed from the same region, and therefore short dummy RNA having the same sequence can be used in either DNA demethylation model. The short dummy RNA will typically be provided from a viral vector, but when a viral vector is not used, it may be provided by creating a recombinant plant such that it is transcribed from a sequence inserted downstream from the promoter of the plant expression vector. However, the supply rate of the short dummy RNA is thought to be much higher when supply is from a viral vector.

The upper limit for the short dummy RNA length may be 200 bp or shorter, 190 bp or shorter, 180 bp or shorter, 170 bp or shorter, 160 bp or shorter, 150 bp or shorter, 140 bp or shorter, 130 bp or shorter, 120 bp or shorter, 110 bp or shorter, 100 bp or shorter, 90 bp or shorter, 80 bp or shorter, 70 bp or shorter, 60 bp or shorter or 50 bp or shorter, for example, and the lower limit for the short dummy RNA length may be 19 bp or longer, 20 bp or longer, 21 bp or longer, 22 bp or longer, 23 bp or longer, 24 bp or longer, 25 bp or longer, 30 bp or longer, 35 bp or longer, 40 bp or longer or 45 bp or longer, for example. The short dummy RNA length will typically be 19 bp to 200 bp, and is preferably 20 bp to 80 bp and most preferably 20 bp to 50 bp.

The target DNA is not particularly restricted so long as it is DNA contributing to expression of the desired trait in plants and controlled by methylation, and as an example, it may be a promoter that controls a gene for expression of a desired trait for plant cells. Examples of desired traits include properties observed in the outer appearance (form) such as shape, color and size, and observable physiological properties such as the flowering period, as well as other physiological properties such as resistance to pathogenic organisms and temperature, but preferred properties are those relating to accumulation of functional components (metabolic components) in plants. The gene that expresses the desired trait is therefore preferably a gene coding for the amino acid sequence of an enzyme that controls synthesis or accumulation of a plant-derived functional component.

Examples of plant-derived functional components include acetylenes, thiophenes, glycosides, glucosinates, purines, pyrimidines, alkaloids, phenolics (such as quinones), essential oils, vitamins, terpenoids (such as iridoids, sesquiterpenes, diterpenoids and triterpenoids), lignans and flavonoids.

Introduction of the short dummy RNA into plant cells is preferably carried out using recombination technology, or using a transient expression system for convenience and speed. Examples are the plant virus vector method, agroinfiltration method, magnICON^{®} system and particle gun method, as well as combinations thereof.

In the plant virus vector method, cDNA of a plant virus genome with the target DNA inserted is transcribed *in vitro,* the obtained RNA is inoculated as vector into a plant for infection, and the proliferation potency and systemic transferability of the virus itself is used for expression of the target gene in the plant. This method expresses the target gene by utilizing the auto-replicating ability of the virus, and therefore the expression level of the target gene in each plant cell can be increased by using a vector based on CMV or TMV, which have especially high proliferation potency.

In agroinfiltration, a culture solution of an *Agrobacterium* microbe transformed with a target gene-inserted T-DNA vector is introduced into plant tissue by a physical method (such as syringe injection or depressurized permeation), to infect the plant, thereby causing transient expression of the target gene in the plant. With this method it is possible to translocate and systemically infect a plant body with highly infectious *Agrobacterium* using a physical method (injection or permeation), thus causing uniform expression of the target gene throughout the tissue of the plant. *Agrobacterium* is a general name for a pathogenic group of bacteria among *Rhizobium,* a gram-negative soil bacterium genus, with examples of *Agrobacterium* including *Agrobacterium tumifaciens* which is associated with root carcinoma disease. When an *Agrobacterium*-derived vector is used for transient expression of an exogenous gene in a plant, the vector is usually introduced into the plant tissue by a physical method (syringe injection or depressurized permeation), to infect the plant. Since an *Agrobacterium*-derived vector has strong infectivity by acting on the T-DNA region, it can be used for infection in a uniform systemic manner in the plant body by a physical method (injection or permeation) to allow an exogenous gene to be expressed uniformly and evenly throughout the tissue of the plant.

In a magnICON^{®} system, TMV or PVX genomic cDNA having a target gene inserted is introduced into a T-DNA vector, and a culture solution of the *Agrobacterium* transformed with the obtained T-DNA vector is introduced into plant tissue by a physical method (syringe injection or depressurized permeation) to infect the plant, thereby causing transient expression of the target gene in the plant. In other words, the vector can be spread out systemically through the plant body by physical means (injection or permeation) to produce infection, thereby causing expression of the target gene throughout the entire tissue of the plant. Since the target gene is expressed by utilizing the auto-replicating ability of a virus (TMV or PVX), it is possible to increase the expression level of the target gene in each plant cell. This method, therefore, combines the advantages of the plant virus vector method and agroinfiltration method.

In addition, a nucleic acid molecule comprising a sequence corresponding to the RNA2 genome of cucumber mosaic virus (CMV) in which all or part of the gene coding for the 2b protein has been replaced with an exogenous gene, in functional combination with the T-DNA sequence of *Agrobacterium,* may be introduced into a host plant that functionally expresses the RNA1 genome and RNA3 genome of a different CMV, as well as protein 2b, and the plant may be cultivated to express the exogenous gene, thereby allowing the exogenous gene to be spread and expressed uniformly in all of the cells of the plant body while also increasing the efficiency of expression of the exogenous gene in the cells, so that high expression can be achieved throughout the entire plant body (PTL 1). This method can be employed as a suitable technique for the magnICON^{®} system using TMV or PVX, and makes it possible to increase the degree of freedom for types of host plants and introducible exogenous gene sizes.

In the particle gun (or particle bombardment) method, fine particles of a metal such as gold or tungsten coated with DNA or a vector are ejected at high speed to introduce the target DNA into cells.

The vector used to introduce the short dummy RNA is not particularly restricted so long as the short dummy RNA can be introduced into plant cells, but it will typically be a plant virus vector or T-DNA vector, and most preferably a plant virus vector. Plant virus vectors are not particularly restricted so long as they can infiltrate into the nucleus of plant cells, and they include vectors derived from single-stranded RNA viruses such as Tobacco mosaic virus (TMV), Cucumber mosaic virus (CMV), Potato X virus (PVX) and Clover yellow vein virus (ClYVV), single-stranded DNA viruses such as Bean yellow dwarf virus (BeYDV), Beet curly top virus (BCTV), Cabbage leaf-curl virus (CaLCuV), Wheat dwarf virus (WDV) and Tomato yellow leaf curl China virus (TYLCCNV), and double-stranded DNA viruses such as Cauliflower mosaic virus (CaMV). Particularly preferred among these are vectors derived from the RNA virus Cucumber mosaic virus (CMV), among which CMV-A1 vector is an example.

The species of plants to which the methods and expression systems of the invention may be applied are not particularly restricted, but typical plants include those of Poaceae, Leguminosae, Brassicaceae, Compositae, Solanaceae, Rosaceae, Cucurbitaceae and Convolvulaceae. Examples of preferred plants include alfalfa, barley, green bean, canola, cowpea, cotton, corn, clover, lotus, lentil, lupin, millet, oat, pea, peanut, rice, rye, sweet clover, sunflower, sweet pea, soybean, sorghum, triticale, jicama, velvet bean, horse-bean, wheat, wisteria, nut plants, thale cress, redtop grass, Welsh onion, snapdragon, Dutch celery, peanut, asparagus, *Atropa,* wild oat, thorny bamboo, rape, bromegrass, rurimagaribana, camellia, cannabis, capsicum, chickpea, goosefoot, chicory, citrus, coffee tree, juzudama, cucumber, pumpkin, bermudagrass, dactylis, jimsonweed, digitalis, yam, oil palm, ooshiba, fescue, strawberry, geranium, soybean, sunflower, Hemerocallidoideae, Para rubber plant, henbane, sweet potato, lettuce, *Lens culinaris,* lily, flax, ryegrass, lotus, tomato, marjoram, apple, mango, manihot, burr medic, African toadflax, tobacco, onobrychis, geranium, Chinese fountain grass, petunia, phleum, meadow grass, cherry flower, buttercup, radish, currant, castor bean, raspberry, sugarcane, *Salpiglossis,* senecio, setaria, white mustard, eggplant, sorghum, buffalo grass, cacao, clover, *Trigonella caerulea* and grape.

The plant cells may be in any form, such as non-isolated cells present in the plant body, or cultured cells from tissue culture isolated from the plant body. In the case of cultured cells, they may be differentiated cells, dedifferentiated cells, or redifferentiated cells.

### [3. Method for creating plant with desired trait]

The method for inhibiting methylation of target DNA may be used to inhibit methylation of DNA that contributes to expression of a desired trait for a plant, in order to create a plant having the desired trait. According to another aspect of the invention, therefore, there is provided a method for creating a plant with a desired trait, wherein the method comprises using the method described above to inhibit methylation of DNA that contributes to expression of a desired trait for plants.

### [4. Method for creating plant-derived functional component]

The method for inhibiting methylation of target DNA may also be used to inhibit methylation of DNA that contributes to synthesis and accumulation of a plant-derived functional component in order to produce the plant-derived functional component in a convenient and rapid manner. Therefore, also described herein is a method for producing a plant-derived functional component wherein the method described above is used to inhibit methylation of DNA that contributes to synthesis and accumulation of the plant-derived functional component in order to accumulate the functional component in the plant cells, and the functional component is recovered from the plant cells, as well as an expression system for accumulation of a plant-derived functional component, wherein the expression system includes (A) a plant body or plant cells that produce scaffold RNA produced by transcription of DNA and a siRNA-AGO4 complex that contribute to synthesis or accumulation of the plant-derived functional component, in an RNA-directed DNA methylation mechanism, and (B) short dummy RNA comprising the sequence complementary to the siRNA incorporated in the siRNA-AGO4 complex or a sequence complementary to at least a portion of the scaffold RNA.

The present invention will now be explained in greater detail by the following examples. It is to be understood, however, that the invention is not restricted in any way to the examples, and various modifications thereof may be implemented.

### EXAMPLES

### [Experiment 1] Creation of short dummy RNA construct

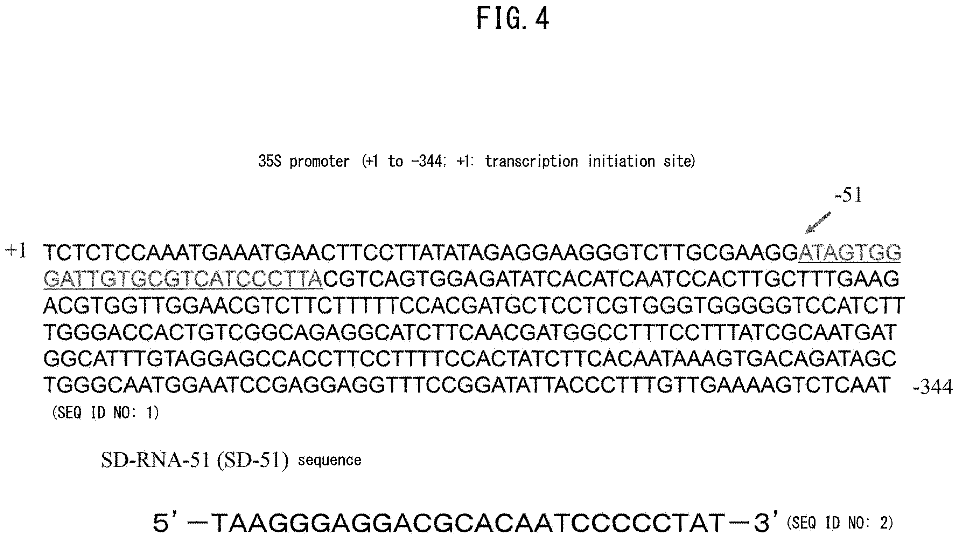

Fig. 4 shows the sequence of the 35S promoter (SEQ ID NO: 1) that synthesizes siRNA homologous with a short dummy RNA construct (underlined portion). Assuming the use of a DNA demethylation model based on capturing siRNA-AGO4 complex (Fig. 2) and/or a DNA demethylation model based on blocking binding between the scaffold RNA and siRNA-AGO4 by short dummy RNA (Fig. 3), oligo DNA 35S (-)-sdRNA-(-51)-5St(5'-CGAGGCCTTAAGGGATGACGCACAATCCCACTA-3': SEQ ID NO: 3) and 35S (-)-sdRNA-(-51)-3 Ml(5'-CGCACGCGTATAGTGGGATTGTGCGTCATCCCTTA-3': SEQ ID NO: 4) with StuI and MluI restriction sites added at the 5'-ends, respectively, were synthesized for cloning of SD-51 (5'-TAAGGGAGGACGCACAATCCCCCTAT-3': SEQ ID NO: 2) which expresses siRNA homologous to the sequence, and were used to produce double-stranded DNA by PCR. This was incorporated into the infectious cDNA clone plasmid cloning site of CMV-A1 vector (the StuI and MluI restriction sites) by a common method.

### [Experiment 2] Inoculation of short dummy RNA construct

### (1) First generation inoculated with short dummy RNA (SD-51)

N. benthamiana line 208RED created by the present inventors was used as the material for this experiment. The preparation details and characteristics of 208RED are as follows. First, N. benthamiana line 16c (see NPL 4) which emits GFP fluorescence by the introduced GFP gene was infected with CMV-A1 vector (see NPL 5) having a homologous sequence to 35S promoter linked to the GFP gene. Infection of the CMV-A1 vector causes cytosine methylation within the 35S promoter sequence via the RdDM pathway, resulting in induction of transcriptional gene silencing (TGS) of the GFP gene, and loss of GFP fluorescence. Individuals maintaining TGS of the GFP gene among the self-pollinated progeny of infected individuals were selected out during each generation, and a line stably maintaining TGS throughout the third self-pollinated generation (S3) was used in this experiment as line 208RED.

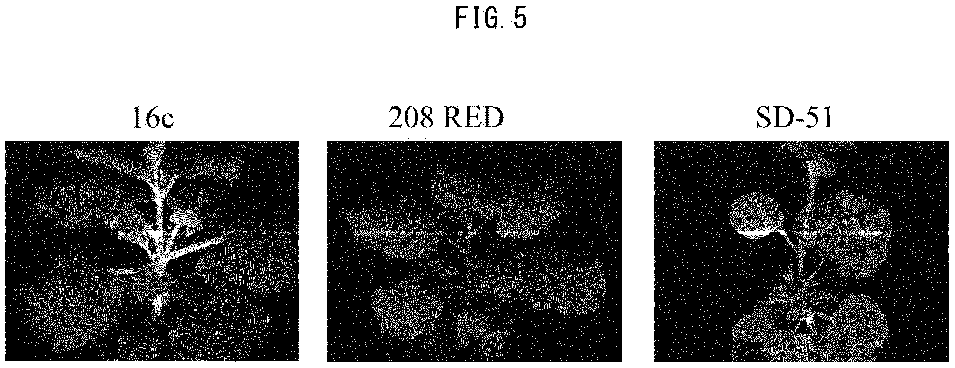

After inoculating the CMV-A1 vector including the short dummy RNA (SD-51) construct in 208RED lacking GFP fluorescence (CMV-A1-SD-51), GFP fluorescence was restored in about 30% of the inoculated individuals (Fig. 5). In 208RED that had not been inoculated with CMV-A1-SD-51, fluorescence was not restored in any of the individuals tested in the experiment (Fig. 5). These results suggested that TGS of the GFP gene had been canceled out by the short dummy RNA (SD-51) in individuals with restored fluorescence by CMV-A1-SD-51 infection.

### (2) Progeny inoculated with short dummy RNA (SD-51)

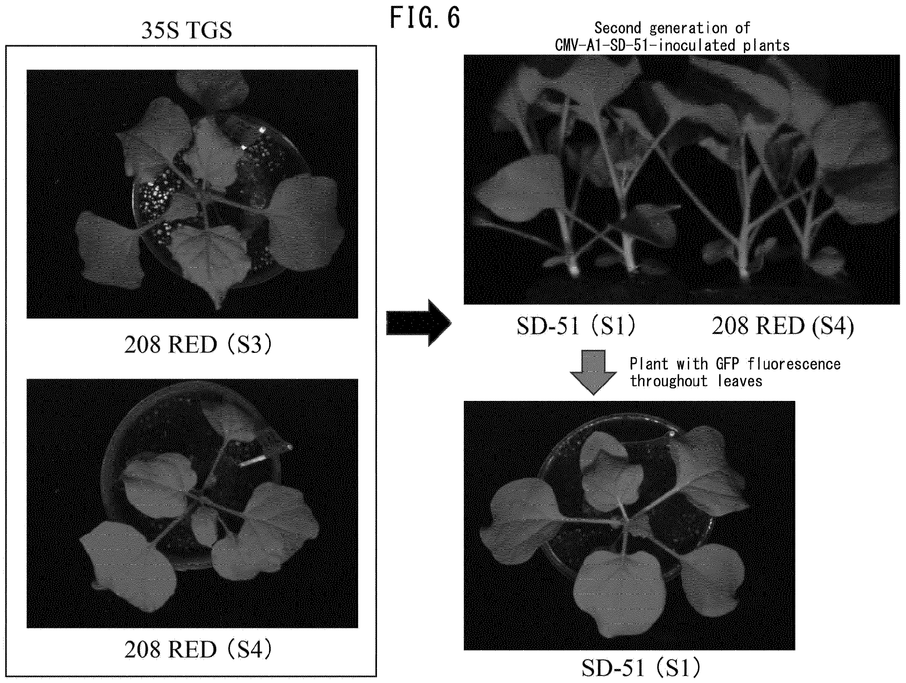

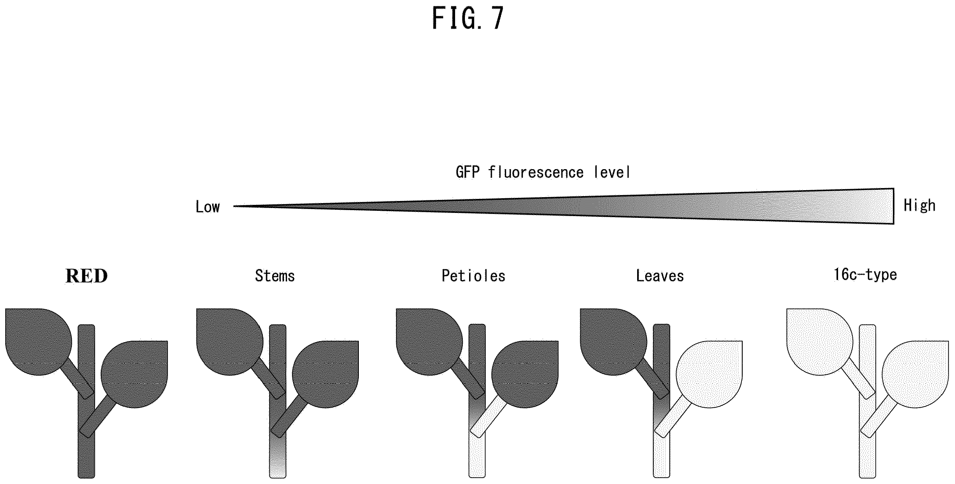

Seeds were taken from the 208RED individuals (16c-type) having fluorescence restored to the same level as 16c (the original GFP expressing N. benthamiana) by CMV-A1-SD-51 infection and grown to the second generation (S1) and subsequent generation (S2), with GFP fluorescence being observed in each generation. Restoration of GFP fluorescence was observed in the stems in both generations, gradually spreading throughout the entire plant body (Fig. 6). The phenotype of GFP fluorescence was classified into 5 groups: RED (no fluorescence), stems, petioles, leaves, and 16c-type (exhibiting the same level of GFP fluorescence as 16c) (Fig. 7).

In individuals S1 and S2 which had restored GFP fluorescence, the GFP fluorescence was gradually restored during growth, with the same level of GFP fluorescence as 16c being exhibited in about 40% of individuals among S2 (Table 1).

**[Table 1]**

| | | GFP fluorescence level | | | | |
|---|---|---|---|---|---|---|
| | Days after inoculation | RED | Stems | Petioles | Leaves | 16c-type |
| SD-51 (S1) | 35 days | 84 | 13 | 1 | 1 | 1 |
| | 50 days | 42 | 15 | 20 | 20 | 3 |
| | 60 days | 40 | 15 | 20 | 20 | 5 |
| SD-51 (S2) | 35 days | 60 | 0 | 10 | 15 | 15 |
| | 50 days | 30 | 0 | 15 | 20 | 20 35 |
| | 60 days | 15 | 5 | 20 | 20 | 40 |

(%) (3) DNA methylation of 35S promoter short dummy RNA (SD-51)-inoculated first generation and second generation.

DNA was extracted from the leaves of individuals of the first generation and second generation of 208RED individuals and 208RED individuals inoculated with CMV-A1-SD-51, using an Illustra DNA Extraction Kit Nucleon Phytopure (GE Healthcare), and the extracted DNA was subjected to bisulfite treatment using an EZ DNA Methylation-Lightning Kit (Zymo Research), converting the non-methylated cytosine to uracil. A TaKaRa EpiTaq HS for bisulfite-treated DNA (TaKaRa) was used for PCR amplification of the 35S promoter region from the bisulfite-treated DNA. The PCR primers used for plus strand detection were 35S-346F-bisuT (5'-ATTGAGAYTTTTYAAYAAAGGGTA-3': SEQ ID NO: 5) and 35S+1A-bisuA (5'-CTCTCCAAATGAAATGAACTTC-3': SEQ ID NO: 6). The PCR primers used for minus strand detection were 35S (-)-5-BS (5'-TTATATAGAGGAAGGGTYTTGYGAAG-3': SEQ ID NO: 7) and 35S (-)-3-BS (5'-CAATTRARACTTTTCAACAAAR-3': SEQ ID NO: 8). The obtained PCR product was ligated to pTAC1 vector using a Dyna Express TA PCR Cloning Kit (Bio Dynamics Laboratory). This was then used for transformation of *E. coli* JM109 Competent Cells (TaKaRa), and the proliferated plasmids were extracted. The extracted plasmids were sequenced by a common method. As a result of comparing the frequency of methylation of cytosine in the 35S promoter region for the CG, CHG (H = A/C/T) and CHH sites, based on sequencing results, it was confirmed that demethylation of cytosine had been induced in SD-51-inoculated individuals at all of the sites (Table 2).

**[Table 2]**

| | Inoculated first generation | | Inoculated second generation | |
|---|---|---|---|---|
| | SD-51 plus strand | SD-51 minus strand | SD-51 (S1) plus strand | SD-51 (S1) minus strand |
| CHH | 0.15 | 0.7 | 0.7 | 1 |
| CHG | 0.3 | 0.3 | 0.7 | 0.8 |
| CG | 0.3 | 0.4 | 0.7 | 0.7 |
| Total | 0.2 | 0.5 | 0.7 | 0.9 |

| | | | | |
|---|---|---|---|---|
| * The numerical values are relative values in the case where the methylation rate of 208RED, which is a TGS control, is 1. | | | | |

[Sequence Listing]

## Claims

1. A method for inhibiting methylation of a target DNA in a plant cell, comprising a step of inhibiting binding of a scaffold RNA produced by transcription of the target DNA with an siRNA-AGO4 complex, in an RNA-directed DNA methylation mechanism,
wherein the step of inhibiting binding of the scaffold RNA with the siRNA-AGO4 complex is accomplished by introducing into the plant cell a short dummy RNA,
wherein the short dummy RNA comprises i) a sequence complementary to the siRNA incorporated in the siRNA-AGO4 complex, or ii) a sequence complementary to the scaffold RNA, and
wherein the length of the scaffold RNA is 20 bp to 50 bp.

2. The method according to claim 1, wherein the short dummy RNA comprises a nucleotide sequence having at least 80% or greater, 85% or greater, 90% or greater, 92% or greater, 94% or greater, 95% or greater, 96% or greater, 97% or greater, 98% or greater or 99% or greater identity with the target DNA.

3. The method according to claim 1 or 2, wherein the target DNA is a promoter that controls a gene expressing a desired trait for the plant cell.

4. The method according to claim 3, wherein the gene that expresses the desired trait is a gene coding for the amino acid sequence of an enzyme that controls synthesis or accumulation of a plant-derived functional component.

5. The method according to any one of claims 1 to 4, wherein the short dummy RNA is introduced into the plant cell by a plant virus vector method, an agroinfiltration method, a magnICON^{®} system or a particle gun method.

6. The method according to any one of claims 1 to 5, wherein the plant cell is a non-isolated cell of a plant body.

7. The method according to any one of claims 1 to 5, wherein the plant cell is a cultured cell.

8. A method for creating a plant with a desired trait, comprising a step of inhibiting methylation of a DNA involved in expression of the desired trait for the plant by using the method according to any one of claims 1 to 7.

9. A method for producing a plant-derived functional component, comprising steps of inhibiting methylation of DNA involved in synthesis or accumulation of a plant-derived functional component by using the method according to any one of claims 1 to 7, thereby causing accumulation of the functional component in a plant cell, and recovering the functional component from the plant cell.

10. An expression system for accumulation of a plant-derived functional component, comprising:
(A) a plant body or a plant cell that produces a scaffold RNA produced by transcription of a DNA involved in synthesis or accumulation of the plant-derived functional component, and siRNA-AGO4 complex, in an RNA-directed DNA methylation mechanism, and
(B) a short dummy RNA comprising a sequence complementary to the siRNA incorporated in the siRNA-AGO4 complex, or a sequence complementary to the scaffold RNA, and
wherein the length of the scaffold RNA is 20 bp to 50 bp.

11. The expression system according to claim 10, wherein the short dummy RNA comprises a nucleotide sequence having at least 80% or greater, 85% or greater, 90% or greater, 92% or greater, 94% or greater, 95% or greater, 96% or greater, 97% or greater, 98% or greater or 99% or greater identity with the DNA involved in synthesis or accumulation of the plant-derived functional component

## Patentansprüche

1. Verfahren zum Hemmen der Methylierung einer Ziel-DNA in einer Pflanzenzelle, umfassend einen Schritt, bestehend im Hemmen des Bindens einer durch Transkription der Ziel-DNA erzeugten Gerüst-RNA an einen siRNA-AGO4-Komplex in einem RNA-gesteuerten DNA-Methylierungs-Mechanismus,
wobei der Schritt, bestehend im Hemmen des Bindens der Gerüst-RNA an den siRNA-AGO4-Komplex, durch Einbringen einer kurzen Dummy-RNA in die Pflanzenzelle erreicht wird,
wobei die kurze Dummy-RNA i) eine zu der in den siRNA-AGO4-Komplex eingebrachten siRNA komplementäre Sequenz oder ii) eine zu der Gerüst-RNA komplementäre Sequenz umfasst, und
wobei die Länge der Gerüst-RNA 20 bp bis 50 bp beträgt.

2. Verfahren gemäß Anspruch 1, wobei die kurze Dummy-RNA eine Nukleotidsequenz mit 80 % oder mehr, 85 % oder mehr, 90 % oder mehr, 92 % oder mehr, 94 % oder mehr, 95 % oder mehr, 96 % oder mehr, 97 % oder mehr, 98 % oder mehr oder 99 % oder mehr Identität mit der Ziel-DNA umfasst.

3. Verfahren gemäß Anspruch 1 oder 2, wobei es sich bei der Ziel-DNA um einen Promotor handelt, der ein Gen steuert, welches ein gewünschtes Merkmal für die Pflanzenzelle exprimiert.

4. Verfahren gemäß Anspruch 3, wobei es sich bei dem Gen, welches das gewünschte Merkmal exprimiert, um ein Gen handelt, das die Aminosäuresequenz eines Enzyms kodiert, welches die Synthese oder Anreicherung einer pflanzlichen funktionellen Komponente steuert.

5. Verfahren gemäß einem der Ansprüche 1 bis 4, wobei die kurze Dummy-RNA durch ein Pflanzenvirusvektorverfahren, ein Agroinfiltrationsverfahren, ein magnlCON^{®}-System oder ein Partikelkanonenverfahren in die Pflanzenzelle eingebracht wird.

6. Verfahren gemäß einem der Ansprüche 1 bis 5, wobei es sich bei der Pflanzenzelle um eine nicht isolierte Zelle eines Pflanzenkörpers handelt.

7. Verfahren gemäß einem der Ansprüche 1 bis 5, wobei es sich bei der Pflanzenzelle um eine gezüchtete Zelle handelt.

8. Verfahren zum Erzeugen einer Pflanze mit einem gewünschten Merkmal, umfassend einen Schritt, bestehend im Hemmen der Methylierung einer an der Expression des gewünschten Merkmals für die Pflanze beteiligten DNA unter Verwendung des Verfahrens gemäß einem der Ansprüche 1 bis 7.

9. Verfahren zum Herstellen einer pflanzlichen funktionellen Komponente, umfassend Schritte, bestehend im Hemmen der Methylierung von DNA, die an der Synthese oder Anreicherung einer pflanzlichen funktionellen Komponente beteiligt ist, unter Verwendung des Verfahrens gemäß einem der Ansprüche 1 bis 7, wodurch die Anreicherung der funktionellen Komponente in einer Pflanzenzelle verursacht wird, und im Gewinnen der funktionellen Komponente aus der Pflanzenzelle.

10. Expressionssystem zur Anreicherung einer pflanzlichen funktionellen Komponente, umfassend:
(A) einen Pflanzenkörper oder eine Pflanzenzelle, die eine Gerüst-RNA, welche durch Transkription einer an der Synthese oder Anreicherung der pflanzlichen funktionellen Komponente beteiligten DNA produziert wird, und einen siRNA-AGO4-Komplex in einem RNA-gesteuerten DNA-Methylierungs-Mechanismus produziert, und
(B) eine kurze Dummy-RNA, umfassend eine zu der in den siRNA-AGO4-Komplex eingebrachten siRNA komplementäre Sequenz oder eine zu der Gerüst-RNA komplementäre Sequenz, und
wobei die Länge der Gerüst-RNA 20 bp bis 50 bp beträgt.

11. Expressionssystem gemäß Anspruch 10, wobei die kurze Dummy-RNA eine Nukleotidsequenz mit mindestens 80 % oder mehr, 85 % oder mehr, 90 % oder mehr, 92 % oder mehr, 94 % oder mehr, 95 % oder mehr, 96 % oder mehr, 97 % oder mehr, 98 % oder mehr oder 99 % oder mehr Identität mit der an der Synthese oder Anreicherung der pflanzlichen funktionellen Komponente beteiligten DNA umfasst.

## Revendications

1. Procédé d'inhibition de la méthylation d'un ADN cible dans une cellule de plante, comprenant une étape d'inhibition de la liaison d'un ARN échafaudage produit par transcription de l'ADN cible avec un complexe siARN-AGO4, dans un mécanisme de méthylation de l'ADN dirigée par l'ARN,
dans lequel l'étape d'inhibition de la liaison de l'ARN échafaudage avec le complexe siARN-AGO4 est accomplie par l'introduction dans la cellule de plante d'un ARN leurre court,
dans lequel l'ARN leurre court comprend i) une séquence complémentaire au siARN incorporé dans le complexe siARN-AGO4, ou ii) une séquence complémentaire à l'ARN échafaudage, et dans lequel la longueur de l'ARN échafaudage est de 20 pb à 50 pb.

2. Procédé selon la revendication 1, dans lequel l'ARN leurre court comprend une séquence nucléotidique présentant au moins 80 % ou plus, 85 % ou plus, 90 % ou plus, 92 % ou plus, 94 % ou plus, 95 % ou plus, 96 % ou plus, 97 % ou plus, 98 % ou plus ou 99 % ou plus d'identité avec l'ADN cible.

3. Procédé selon la revendication 1 ou la revendication 2, dans lequel l'ADN cible est un promoteur qui commande un gène exprimant un caractère souhaité pour la cellule de plante.

4. Procédé selon la revendication 3, dans lequel le gène qui exprime le caractère souhaité est un gène codant pour la séquence d'acides aminés d'une enzyme qui commande la synthèse ou l'accumulation d'un composant fonctionnel dérivé de plante.

5. Procédé selon l'une quelconque des revendications 1 à 4, dans lequel l'ARN leurre court est introduit dans la cellule de plante par une méthode par vecteur de virus de plante, une méthode d'agroinfiltration, un système magnlCON^{®} ou une méthode par canon à particules.

6. Procédé selon l'une quelconque des revendications 1 à 5, dans lequel la cellule de plante est une cellule non isolée d'un corps de plante.

7. Procédé selon l'une quelconque des revendications 1 à 5, dans lequel la cellule de plante est une cellule cultivée.

8. Procédé de création d'une plante avec un caractère souhaité, comprenant une étape d'inhibition de la méthylation d'un ADN impliqué dans l'expression du caractère souhaité pour la plante en utilisant le procédé selon l'une quelconque des revendications 1 à 7.

9. Procédé de production d'un composant fonctionnel dérivé de plante, comprenant des étapes d'inhibition de la méthylation d'un ADN impliqué dans la synthèse ou l'accumulation d'un composant fonctionnel dérivé de plante en utilisant le procédé selon l'une quelconque des revendications 1 à 7, permettant ainsi de provoquer l'accumulation du composant fonctionnel dans une cellule de plante, et de récupérer le composant fonctionnel à partir de la cellule de plante.

10. Système d'expression pour l'accumulation d'un composant fonctionnel dérivé de plante, comprenant :
(A) un corps de plante ou une cellule de plante qui produit un ARN échafaudage produit par transcription d'un ADN impliqué dans la synthèse ou l'accumulation du composant fonctionnel dérivé de plante, et un complexe siARN-AGO4, dans un mécanisme de méthylation de l'ADN dirigée par l'ARN, et
(B) un ARN leurre court comprenant une séquence complémentaire au siARN incorporé dans le complexe siARN-AGO4, ou une séquence complémentaire à l'ARN échafaudage, et
dans lequel la longueur de l'ARN échafaudage est de 20 pb à 50 pb.

11. Système d'expression selon la revendication 10, dans lequel l'ARN leurre court comprend une séquence nucléotidique présentant au moins 80 % ou plus, 85 % ou plus, 90 % ou plus, 92 % ou plus, 94 % ou plus, 95 % ou plus, 96 % ou plus, 97 % ou plus, 98 % ou plus ou 99 % ou plus d'identité avec l'ADN impliqué dans la synthèse ou l'accumulation du composant fonctionnel dérivé de plante.
